# EUROPÄISCHE PATENTSCHRIFT

(11) **EP 4 001 943 B1**
(45) Veröffentlichungstag und Bekanntmachung des Hinweises auf die Patenterteilung: **14.05.2025**
(21) Anmeldenummer: 20208815.9
(22) Anmeldetag: 20.11.2020
(51) Int. Cl.: G01R 33/34, A61B 5/055, A61B 5/00, G01R 33/341

(54) **MR-LOKALSPULE UND VERFAHREN ZU DEREN HERSTELLUNG**
MR LOCAL COIL AND METHOD FOR THE PRODUCTION OF SAME
BOBINE LOCALE MR ET SON PROCÉDÉ DE FABRICATION

(43) Veröffentlichungstag der Anmeldung: 25.05.2022
(73) Patentinhaber: Siemens Healthineers AG, 91301 Forchheim (DE)
(72) Erfinder: Strauchmann, Christina, 91330 Eggolsheim (DE)
(74) Vertreter: Siemens Healthineers Patent Attorneys

(56) Entgegenhaltungen:
- DE-A1- 102018 216 365
- DE-U1- 202020 104 846

## Beschreibung

Die Erfindung betrifft eine MR-Lokalspule, eine Magnetresonanzvorrichtung mit einer MR-Lokalspule und ein Verfahren zur Herstellung einer MR-Lokalspule, wie in den Ansprüche 1, 13, und 14 definiert.

In der Medizintechnik zeichnet sich die Bildgebung mittels Magnetresonanz (MR), auch Magnetresonanztomographie (MRT, engl. Magnetic Resonance Imaging, MRI) genannt, durch hohe Weichteilkontraste aus. Hierbei werden mit Hilfe einer Magnetresonanzvorrichtung Anregungspulse in ein Untersuchungsobjekt, welches in der Regel ein Patient ist, eingestrahlt. Dadurch werden im Patienten Magnetresonanzsignale ausgelöst. Die Magnetresonanzsignale werden als Messdaten von der Magnetresonanzvorrichtung empfangen und zur Rekonstruktion von Magnetresonanzabbildungen verwendet.

Oftmals erfolgt der Empfang der Magnetresonanzsignale mit so genannten Lokalspulen (engl. local coils), die oft auch Oberflächenspulen (engl. surface coils) genannt werden. Solche MR-Lokalspulen umfassen üblicherweise eine oder mehrere MR-Antennen und werden während einer Magnetresonanzuntersuchung in unmittelbarer Nähe des Patienten angebracht.

Um die MR-Lokalspule an den Patienten gut anformen zu können, sollte diese möglichst leicht und flexibel sein. Um einen hohen Patientenkomfort zu gewährleisten, sollte die MR-Lokalspule am besten wie eine leichte Decke oder ein Kissen wirken und gleichzeitig das Eindringen von Flüssigkeiten verhindern sowie desinfektionsbeständig sein.

Die Druckschrift DE102018216365A1 offenbart eine flexible MR-Lokalspule mit einer Verbindungseinheit, die ausgebildet ist, zwei Umhüllungsflächen zu verbinden. Die Druckschrift DE202020104846U1 offenbart eine flexible Lokalspule mit einer mittels Ultraschallscheißen erzeugten Schweißnaht.

Um die flexiblen MR-Lokalspulen mit der Magnetresonanzvorrichtung verbinden zu können, haben in der Regel einen Spulenanschluss, etwa in Form eines Steckers, der von außen zugänglich und an einer flexiblen Außenhülle der MR-Lokalspule angeordnet ist. Hier ist eine zuverlässige, dichte Verbindung zwischen der flexibler Außenhülle und dem Spulenanschluss wünschenswert. Gleichzeitig sollte die MR-Lokalspule möglichst leicht zu fertigen sein. Als Aufgabe der vorliegenden Erfindung kann angesehen werden, eine nach außen hin möglichst dichte MR-Lokalspule anzugeben, die zudem mit möglichst geringem Aufwand gefertigt werden kann.

Die Aufgabe wird durch die Merkmale der unabhängigen Ansprüche gelöst. Vorteilhafte Ausgestaltungen sind in den abhängigen Ansprüchen beschrieben.

Demnach wird eine MR-Lokalspule vorgeschlagen, die eine Außenhülle, eine Antennenstruktur und einen Rahmen zum Aufnehmen der Antennenstruktur umfasst. Die Außenhülle ist flexibel ausgebildet und umgibt einen Innenraum. Der Rahmen ist zumindest bereichsweise starr ausgebildet und fest mit der Außenhülle verbunden. Die Antennenstruktur ist in dem Innenraum der Außenhülle angeordnet und wird von dem Rahmen auf Position gehalten.

Die Außenhülle ist flexibel, insbesondere biegbar und/oder in allen Raumrichtung geometrisch anpassungsfähig. Vorteilhafterweise sind dazu keine nennenswerten Kräfte nötig. Vorzugsweise weist die Außenhülle eine Dicke von weniger als 5 mm, insbesondere weniger als 2 mm auf.

Die Außenhülle umschließt einen Innenraum. In dem Innenraum können weitere Komponenten der MR-Lokalspule angeordnet werden. Insbesondere kann die Antennenstruktur zumindest teilweise in dem Innenraum angeordnet sein. Die Antennenstruktur ist vorzugsweise flexibel, insbesondere biegbar und/oder in allen Raumrichtung geometrisch anpassungsfähig. Vorteilhafterweise ist die gesamte MR-Lokalspule zumindest bereichsweise flexibel.

Die zumindest eine MR-Antenne der Antennenstruktur kann beispielsweise eine runde, insbesondere eine ovale oder kreisförmige, Form oder eine Form einer Acht aufweisen. Die zumindest eine MR-Antenne kann beispielsweise auf einem Substrat, insbesondere einer Leiterplatte, aufgebracht sein. Die zumindest eine MR-Antenne können beispielsweise Drähte und/oder koaxiale Kabel umfassen. Vorteilhaftweise können in der MR-Antenne durch Magnetresonanzsignale elektrische Spannungen und/oder Ströme induziert werden.

Der Rahmen ist zumindest bereichsweise starr, insbesondere nicht biegsam und/oder nicht flexibel, ausgebildet. Der Rahmen kann an einer oder mehreren Stellen unterbrochen sein, aber in den Bereichen zwischen den Unterbrechungsstellen starr sein.

Der Rahmen ist fest mit der Außenhülle verbunden. Der Rahmen ist beispielsweise mit der Außenhülle verklebt und/oder verschweißt. Der Rahmen ist vorteilhafterweise dicht, insbesondere flüssigkeitsdicht, mit der Außenhülle verbunden. Vorzugsweise können an der Verbindung des Rahmens zur Außenhülle keine Flüssigkeiten in den Innenraum der Außenhülle eindringen. Vorteilhafterweise ermöglicht der Rahmen eine leichte Montage der Antennenstruktur im Innenraum der Außenhülle.

Gemäß der Erfindung weist der Rahmen mehrere Bruchstellen auf. Im Rahmen des Herstellungsprozesses der MR-Lokalspule weist der Rahmen zunächst lediglich Sollbruchstellen auf, die dann während der Herstellung gebrochen werden, so dass der Rahmen dann mehrere Bruchstellen aufweist. Die Sollbruchstellen im Rahmen könnten beispielsweise als Nuten ausgebildet sein.

Eine weitere Ausführungsform der MR-Lokalspule sieht vor, dass der Rahmen mit der Außenhülle mittels einer Ultraschall-schweißnaht verbunden ist. Mittels einer Ultraschweißnaht können besonders stabile und/oder dichte, insbesondere luft- und wasserdichte, Verbindungen herstellt werden.

Durch Ultraschallschweißen können insbesondere thermoplastische Kunststoffe form- oder stoffschlüssig miteinander verbunden werden. Dazu wird üblicherweise durch mechanische Schwingungen im Kunststoff Reibungswärme erzeugt, so dass die Kunststoffe weich werden, schmelzen und sich verbinden.

Ferner ist der Ultraschallschweißprozess vorteilhafterweise leichter in ein Elektronikfertigungsumfeld zu integrieren als z.B. ein Thermofügeprozess, bei dem durch den höheren und ganzflächigen Wärmeeintrag das Umfeld beeinflusst wird, aber auch die Elektronik geschädigt werden kann. Eine Ultraschallschweißung bringt hingegen gezielt die Wärme zum Verschweißen von Materialien ein.

Eine weitere Ausführungsform der MR-Lokalspule sieht vor, dass die Außenhülle eine Außenschicht aus Kunstleder und/oder ein mit Polyurethan (PU) beschichtetes Gewebe umfasst. Solche Materialien sind vorteilhafterweise besonders widerstandsfähig und/oder leicht zu reinigen und/oder haptisch angenehm. Die zudem, insbesondere innenliegende Schichten umfassen, wie beispielsweise eine Polsterschicht und/oder eine Gleitschicht.

Eine Polsterschicht, z.B. aus Polsterwatte, kann beispielsweise der MR-Lokalspule die Eigenschaft verleihen, dass sie sich weich und/oder kissenähnlich anfühlt. So kann ein Patient die Lokalspule vorteilhafterweise angenehmer wahrnehmen.

Eine Gleitschicht kann beispielsweise der MR-Lokalspule die Eigenschaft verleihen, dass innerhalb der Umhüllung sich befindliche Schichten, wie beispielweise die Antennenstruktur, leichter relativ zueinander bewegen können, da sie sich weniger stark aneinander reiben. Dadurch kann beispielsweise erreicht werden, dass eine bei Verformung der MR-Lokalspule auftretende Rückstellkraft reduziert wird.

Es ist auch denkbar, dass die Außenhülle eine Gleit-Polster-Schicht umfasst, die sowohl gut polstert als auch gut gleitet. Eine solche Gleit-Polster-Schicht kann beispielsweise ein Meshgewebe aufweisen. Ebenso wie separate Gleit- und Polsterschichten kann die Gleit-Polster-Schicht beispielsweise durch die zumindest ein Ultraschall-Schweißnaht direkt mit der Außenschicht verschweißt sein.

Eine weitere Ausführungsform der MR-Lokalspule sieht vor, dass die Außenhülle eine Öffnung zum Einführen der MR-Antenne in die Außenhülle aufweist. Insbesondere weist die Außenhülle nur eine Öffnung zum Einführen der MR-Antenne in die Außenhülle auf. Dadurch, dass nur eine Öffnung vorhanden ist, können möglicherweise undichte Stellen in der Außenhaut reduziert werden.

Eine weitere Ausführungsform der MR-Lokalspule sieht vor, dass der Rahmen an der Öffnung angrenzt. Der Rahmen kann als Hilfe zum Einbringen der Antennenstruktur in das Innere der Außenhaut dienen. Durch eine mögliche Anordnung direkt angrenzend an der Öffnung kann das Einbringen der Antennenstruktur in das Innere der Außenhaut besonders leicht erfolgen.

Eine weitere Ausführungsform der MR-Lokalspule sieht vor, dass die Außenhülle eine flächige Oberseite aufweist, wobei die Außenhülle gegenüber der Oberseite eine flächige Unterseite aufweist, wobei die Oberseite und die Unterseite durch mehrere Stirnseiten verbunden sind, wobei die Öffnung an einer der mehreren Stirnseiten angebracht ist.

Zwischen der Oberseite und der Unterseite der Außenhülle befindet sich vorzugsweise das Innere der Außenhülle. Die mehreren Stirnseiten bilden vorzugsweise jeweils eine Kante der MR-Lokalspule. An zwei aufeinanderstoßen Kanten kann eine Ecke ausgebildet sein. Vorzugsweise erstreckt sich die Öffnung lediglich über einen Teil der Stirnseite bzw. der Kante der MR-Lokalspule. Vorzugsweise beträgt die Erstreckung der Öffnung weniger als die Hälfte der Länge Stirnseite bzw. der Kante der MR-Lokalspule. Mithin ist die Öffnung vorteilhafterweise klein dimensioniert, so dass das Risiko einer Undichtigkeit der MR-Lokalspule reduziert werden kann.

Eine weitere Ausführungsform der MR-Lokalspule sieht vor, dass die mehreren Stirnseiten eine umlaufend einheitliche Außennaht, insbesondere eine Ultraschallschweißnaht, aufweisen. Insbesondere kann die Außennaht entlang der Kanten und Ecken der MR-Lokalspule verlaufen. Vorzugsweise ist die Außennaht über den gesamten Bereich der Ecken und Kanten der MR-Lokalspule mit Ausnahme des Bereichs der Öffnung einheitlich. Dadurch kann die MR-Lokalspule als besonders angenehm und wertig empfunden werden.

Eine weitere Ausführungsform der MR-Lokalspule sieht vor, dass der Rahmen eine Führungsstruktur aufweist, welche mit einer Gegenstruktur der Antennenstruktur so zusammenwirkt, dass die Antennenstruktur innerhalb der Außenhülle auf Position gehalten wird.

Ferner kann die Führungsstruktur bei der Herstellung der MR-Lokalspule dazu dienen, die Antennenstruktur definiert in das Innere der Außenhülle zu bringen. Die Führungsstruktur kann beispielsweise eine Nut und/oder eine Zunge umfassen, entlang der vorzugsweise die Antennenstruktur bei der Herstellung der MR-Lokalspule in das Innere der Außenhülle eingeführt werden kann.

Eine weitere Ausführungsform der MR-Lokalspule sieht vor, dass die Antennenstruktur eine Nut und/oder eine Zunge aufweist, durch welche die Antennenstruktur im Innenraum der Außenhülle auf Position gehalten wird. Beispielsweise kann in eine Nut der Antennenstruktur eine Zunge der Führungsstruktur eingreifen. Es ist auch denkbar, dass eine Zunge der Antennenstruktur in eine Nut der Führungsstruktur eingreift.

Vorzugsweise umfasst das Auf-Position-Halten der Antennenstruktur, dass eine Verschiebung der Antennenstruktur entlang zumindest einer Richtung parallel zur Oberseite und/oder Unterseite der Außenhülle verhindert werden.

Eine weitere Ausführungsform der MR-Lokalspule sieht vor, dass die MR-Lokalspule einen Deckel umfasst, der im Bereich des Rahmens lösbar an der Außenhülle befestigt ist.

Vorzugsweise ist der Deckel ausgebildet, den Innenraum der Außenhülle zu schützen, insbesondere abzudichten. Eine lösbare Befestigung des Rahmens an der Außenhülle kann beispielsweise mit Hilfe von Schrauben erfolgen.

Eine weitere Ausführungsform der MR-Lokalspule sieht vor, dass der Rahmen durch den Deckel geklemmt wird. Vorteilhafterweise wird dadurch eine besonders gute Abdichtung des Innenraums der Außenhülle erreicht.

Ferner wird eine Magnetresonanzvorrichtung mit zumindest einer MR-Lokalspule, wie sie vorab beschrieben wurde. Die Vorteile der vorgeschlagenen MR-Lokalspule entsprechen im Wesentlichen den Vorteilen der Magnetresonanzvorrichtung. Bei der Beschreibung der MR-Lokalspule erwähnte Merkmale, Vorteile oder alternative Ausführungsformen können auch auf die Magnetresonanzvorrichtung übertragen werden.

Ferner wird ein Verfahren zur Herstellung einer der vorab beschriebenen MR-Lokalspulen vorgeschlagen, wobei der Rahmen an den mehreren Bruchstellen gebrochen und die Antennenstruktur entlang der Bruchstellen in die Außenhülle eingeführt wird.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: eine Magnetresonanzvorrichtung mit einer MR-Lokalspule,
- Fig. 2: eine MR-Lokalspule in einer perspektivischen Darstellung,
- Fig. 3: einen Querschnitt durch eine MR-Lokalspule,
- Fig. 4: eine Außenhülle mit einem Rahmen zum Aufnehmen einer Antennenstruktur,
- Fig. 5: einen Rahmen zum Aufnehmen einer Antennenstruktur,
- Fig. 6: eine Detailansicht einer Außenhülle mit einem Rahmen,
- Fig. 7: ein Blockdiagramms eines Verfahrens zur Herstellung einer MR-Lokalspule.

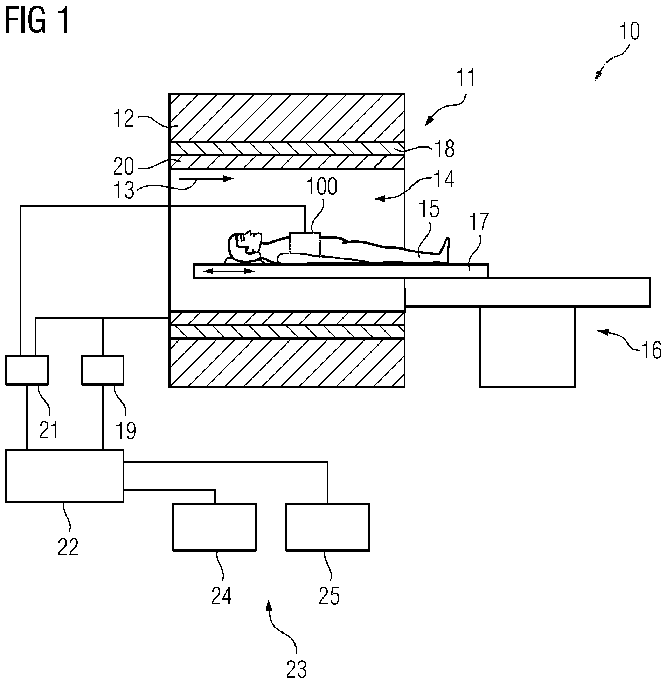

In Fig. 1 ist eine Magnetresonanzvorrichtung 10 schematisch dargestellt. Die Magnetresonanzvorrichtung 10 umfasst eine Magneteinheit 11, die einen Hauptmagneten 12 zu einem Erzeugen eines starken und insbesondere zeitlich konstanten Hauptmagnetfelds 13 aufweist. Zudem umfasst die Magnetresonanzvorrichtung 10 einen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20, welche im vorliegenden Ausführungsbeispiel als fest in die Magnetresonanzvorrichtung 10 integrierte Körperspule ausgebildet ist. Die Hochfrequenzantenneneinheit 20 ist zu einer Anregung von Atomkernen, die sich in dem von dem Hauptmagneten 12 erzeugten Hauptmagnetfeld 13 einstellt, ausgelegt. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in einen Untersuchungsraum ein, der im Wesentlichen von einem Patientenaufnahmebereich 14 der Magnetresonanzvorrichtung 10 gebildet ist. Die Hochfrequenzantenneneinheit 20 ist weiterhin zum Empfang von Magnetresonanzsignalen ausgebildet.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Gradientenechosequenz.

Zudem umfasst die Magnetresonanzvorrichtung 10 eine flexible MR-Lokalspule 100, die unmittelbar am Patienten 15 angeordnet ist. Die MR-Lokalspule 100 umfasst zumindest eine Magnetresonanzantenne und ist ausgebildet, mit der zumindest einen Magnetresonanzantenne Magnetresonanzsignale zu empfangen. Es ist aber auch denkbar, dass sie wie die Hochfrequenzantenneneinheit 20 auch zum Senden von hochfrequenten Magnetresonanzsequenzen ausgebildet ist. Die empfangenen Magnetresonanzsignale werden an die Hochfrequenzantennensteuereinheit 21 übertragen.

Zudem umfasst die Systemsteuereinheit 22 eine nicht näher dargestellte Auswerteeinheit zu einer Auswertung von der Magnetresonanzsignale, die während der Magnetresonanzuntersuchung erfasst werden. Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzabbildungen können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

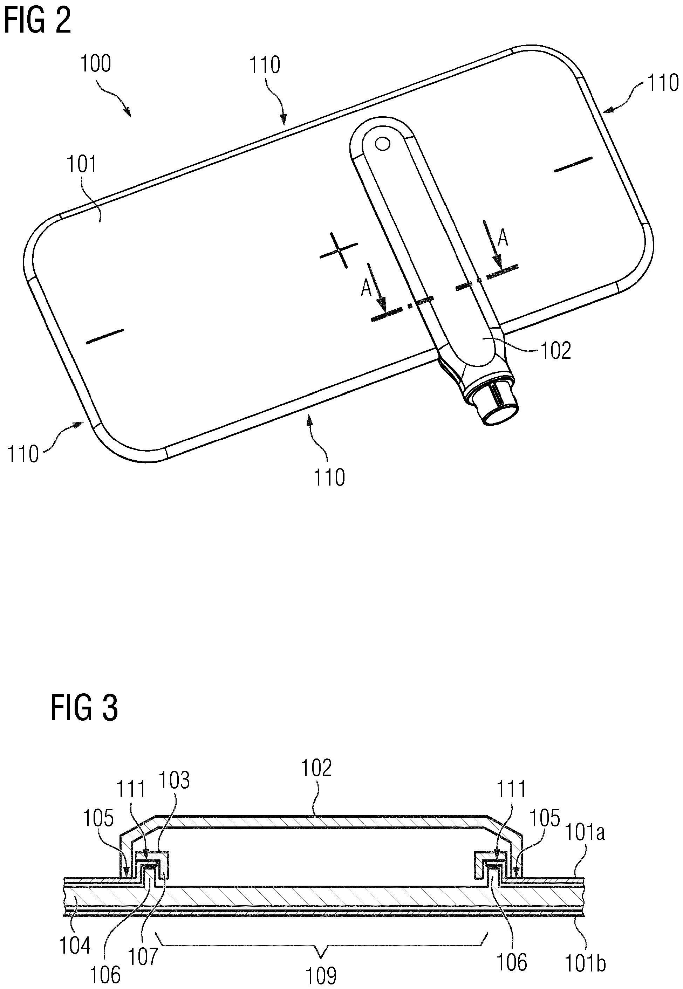

Fig. 2 zeigt eine beispielhafte MR-Lokalspule 100 mit einer Außenhülle 101 und einem Deckel 102. Die Außenhülle 101 umfasst beispielsweise ein Kunstleder und/oder ein PUbeschichtetes Gewebe oder besteht daraus. Der hier angedeutete Querschnitt Schnitt A-A ist in Fig. 3 dargestellt.

Die Außenhülle 101 weist eine flächige Oberseite 101a und eine gegenüberliegende flächige Unterseite 101b auf. Dazwischen liegt ein Innenraum der Außenhülle bzw. der MR-Lokalspule. An vier in Fig. 2 dargestellten Stirnseiten ist die Oberseite 101a mit der Unterseite 101b verbunden.

Ferner umfasst die MR-Lokalspule einen Rahmen 103, der an den Flächen 111 fest mit der Oberseite 101a der Außenhülle 101 verbunden ist, und eine Antennenstruktur 104, die zumindest eine MR-Antenne umfasst und in dem Innenraum der Außenhülle angeordnet ist. Die Verbindung an den Flächen 111 kann beispielsweise durch einen Ultraschallschweißen hergestellt werden, so dass der Rahmen 103 mit der Außenhülle 101 mittels einer Ultraschallschweißnaht verbunden ist

Der Rahmen 103 ist ausgebildet, die Antennenstruktur 104 aufzunehmen und hält sie auf Position. Dazu umfasst der Rahmen eine Führungsstruktur 107 und die Antennenstruktur 104 eine entsprechende Gegenstruktur 106; die Führungsstruktur 107, hier in Form einer Zunge, wirkt mit der Gegenstruktur 106, hier in Form einer Zunge, so zusammen, dass die Antennenstruktur 104 im Innenraum der Außenhülle 101 auf Position gehalten wird. Beispielsweise kann die Antennenstruktur 104 somit nicht seitlich verrutschen.

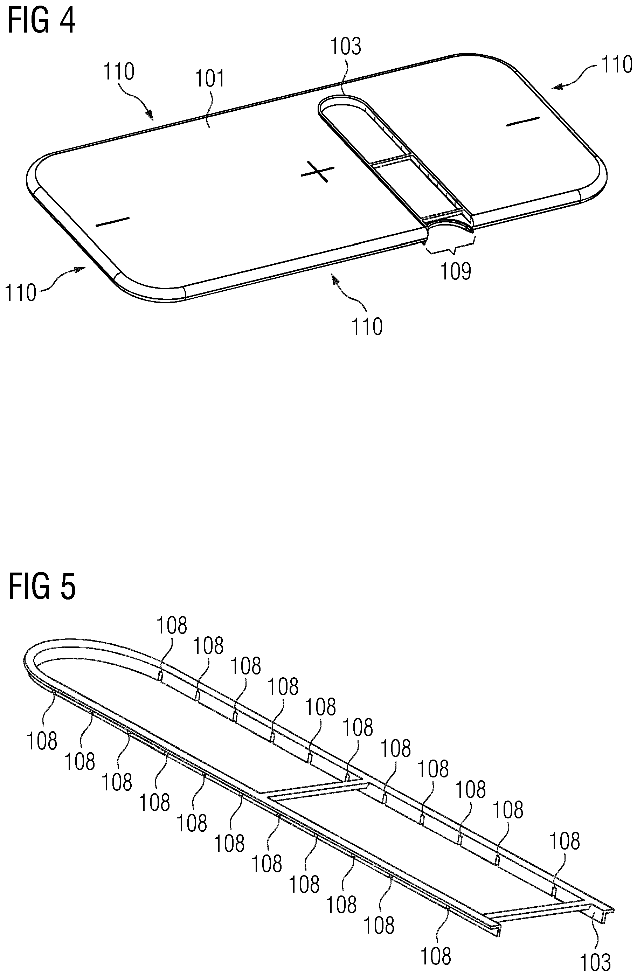

Der Rahmen 103 ist in den Fig. 4 und 5 näher dargestellt. Er umfasst während des Fertigungsprozesses zunächst noch Sollbruchstellen 108, welche im Laufe des Fertigungsprozesses jedoch gebrochen werden, so dass er mehrere Bruchstellen aufweist. Somit ist der Rahmen 103 zwischen den Bruchstellen bereichsweise starr ausgebildet.

Der in Fig. 5 und 5 dargestellte Rahmen 103 weist zwei Querstege auf. Diese können vorteilhafterweise den Rahmen 103 versteifen, so dass er während der Herstellung der MR-Lokalspule 100, insbesondere während eines Anschweißens des Rahmens 103 an die Außenhülle 101, besser gehandhabt werden kann. Vorzugsweise weisen die beiden Querstege an ihren Enden, also am Übergang zum restlichen Rahmen, weitere Sollbruchstellen auf. Durch Brechen des Rahmens 103 an diesen Sollbruchstellen können die Querstege leicht entfernt werden, wodurch eine besonders große Öffnung zum Einbringen der Antennenstruktur 104 entsteht.

Wie in Fig. 3 und 4 dargestellt, weist die Außenhülle 101 an einer der mehreren Stirnseiten 101 eine Öffnung 109 zum Einführen der Antennenstruktur 104 in den Innenraum der Außenhülle 101 auf. Der Rahmen 103 grenzt hierbei an die Öffnung 109 an. Jenseits der Öffnung 109 weisen die Stirnseiten 110 eine umlaufend einheitliche Außennaht auf. Die Außennaht kann beispielsweise eine Ultraschallschweißnaht sein.

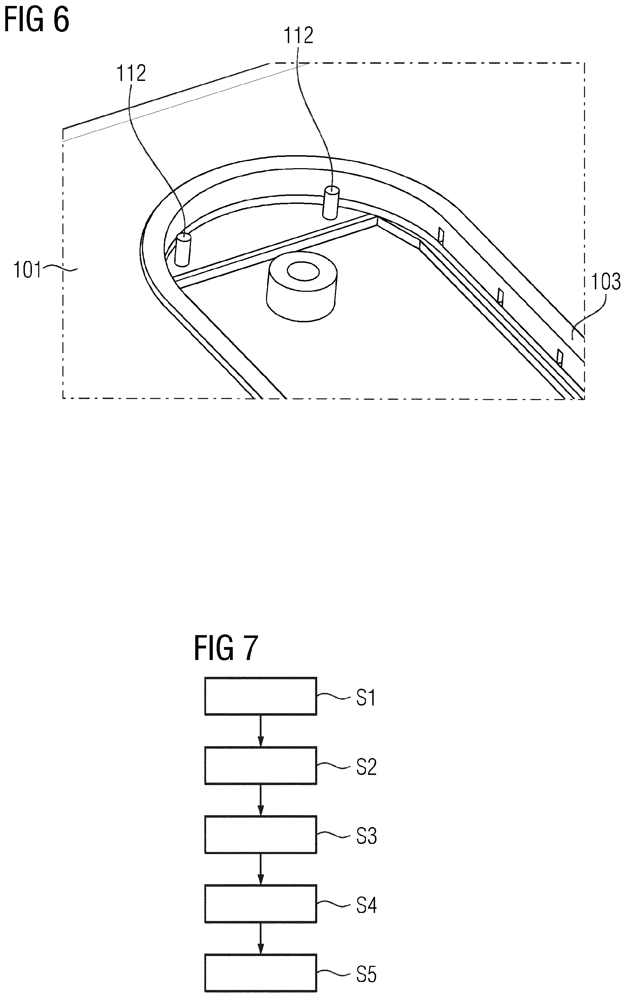

Der in Fig. 2 und 3 dargestellte Deckel 102 ist im Bereich des Rahmens 103 lösbar an der Außenhülle befestigt. Hierbei wird der Rahmen 103 durch den Deckel 102 geklemmt. Fig. 7 zeigt ein Ablaufdiagramm eines Verfahrens zur einer MR-Lokalspule.

Mittels Ultraschallschweißen wird in S1 die Außenhülle 101, insbesondere einschließlich verschiedener Futterstoffe, erzeugt. Hierbei entsteht im sichtbaren Bereich der MR-Lokalspule eine komplett geschlossene Außennaht oder eine Außennaht, die lediglich im Bereich der Gehäuseabdeckung unterbrochen ist, siehe Fig. 4. Es ist auch denkbar, den Schritt S1 später im Herstellungsprozess durchzuführen, z.B. erst nach S2.

Im Bereich des später montierten Deckels wird in S2 der Rahmen 103 mittels Ultraschallschweißen die Außenhülle 101, insbesondere einschließlich verschiedener Futterstoffe und/oder Polsterschichten und/oder Gleitschichten, angeschweißt, wodurch die verschiedenen Materialien fest mit dem Rahmen verbunden sind. Der Rahmen 103 ist so gestaltet, dass er so viel Fläche wie für den Schweißprozess erforderlich, zur Verfügung stellt und gleichzeitig so filigran wie möglich ist.

Nachdem der Rahmen 103 fest mit der Außenhülle 101 verschweißt ist, wird der Rahmen 103 in S3 an seinen Sollbruchstellen im angeschweißten Zustand in mehrere Einzelteile zerbrochen. Insbesondere können etwaige stabilisierende Querstege des Rahmens 103 entfernt werden. Hierdurch entsteht trotz seiner bereichsweisen starren Ausführung eine flexible definierte Öffnung zum Innenraum der Außenhülle 101. Je mehr Sollbruchstellen der Rahmen 103 hat, desto flexibler ist die Öffnung.

Durch diese Öffnung wird in S4 die vorbereitete Antennenstruktur 104 entlang der Bruchstellen in die Außenhülle 101 eingefädelt. Die eingefädelte Antennenstruktur 104 enthält Gegenstücke 106 und 112, die am definiert gebrochene Rahmen 103 anliegen, siehe Fig. 3 bzw. Fig. 6.

Anschließend wird in S5 der Deckel 102 aufgeschraubt und dadurch der kontrolliert gebrochene Rahmen 103 definiert geklemmt. Durch den Rahmen 103 wird so sichergestellt, dass die Außenhülle 101 nicht aus der Klemmung herausrutschen kann.

Wie in Fig. 3 gezeigt, wird durch das Aufschrauben des Deckels 102 die Außenhülle 101 definiert im Kantenbereich gequetscht und so zusätzlich eine Dichtung realisiert. Zusammenfassend lässt sich feststellen, dass bei der vorgeschlagene MR-Lokalspule 100 vorteilhafterweise keine zusätzliche Öffnung zur Einbringung der Antennenstruktur 104 in die Außenhülle 101 benötigt wird, die nachträglich in einem weiteren Schritt wieder, etwa durch Ultraschallschweißen oder Kleben, verschlossen werden müsste.

Ein weiterer Vorteil kann dadurch gegeben sein, dass das finale Produkt eine einheitliche Außennaht aufweist und insbesondere kein Übergang in der Naht, insbesondere bei einem Übergang von innenliegender Naht zu außenliegender Naht, erforderlich und somit auch nicht von außen sichtbar ist.

Zusätzlich ist vorteilhafterweise eine solche Naht robuster als eine etwaige geteilte Außennaht, da eine Schließnaht die restliche Außennaht perfekt treffen müsste. Die einheitliche Außennaht lässt das Produkt wertiger erscheinen und ein zusätzlicher Arbeitsschritt entfällt.

Es wird abschließend noch einmal darauf hingewiesen, dass es sich bei der vorhergehend detailliert beschriebenen MR-Lokalspule 100, der Magnetresonanzvorrichtung 10 und dem Verfahren lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

## Patentansprüche

1. MR-Lokalspule (100) umfassend,
- eine Außenhülle (101), die flexibel ausgebildet ist und einen Innenraum umgibt,
- einen Rahmen (103) zum Aufnehmen einer Antennenstruktur (104), der zumindest bereichsweise starr ausgebildet und fest mit der Außenhülle (101) verbunden ist,
- eine Antennenstruktur (104) mit zumindest einer MR-Antenne, die in dem Innenraum der Außenhülle (101) angeordnet ist und von dem Rahmen (103) auf Position gehalten wird,
**dadurch gekennzeichnet, dass** der Rahmen (103) mehrere aus einem Brechen von Sollbruchstellen (108) resultierende Bruchstellen aufweist.

2. MR-Lokalspule (100) nach Anspruch 1,
wobei der Rahmen (103) mit der Außenhülle (101) mittels einer Ultraschallschweißnaht verbunden ist.

3. MR-Lokalspule (100) nach einem der vorangehenden Ansprüche,
wobei die Außenhülle (101) ein Kunstleder und/oder ein PUbeschichtetes Gewebe umfasst.

4. MR-Lokalspule (100) nach einem der vorangehenden Ansprüche,
wobei die Außenhülle (101) eine Öffnung zum Einführen der MR-Antenne in den Innenraum der Außenhülle aufweist.

5. MR-Lokalspule (100) nach Anspruch 4,
wobei der Rahmen (103) an der Öffnung (109) angrenzt.

6. MR-Lokalspule (100) nach Anspruch 4 oder 5,
wobei die Außenhülle (101) eine flächige Oberseite (101a) aufweist,
wobei die Außenhülle (101) gegenüber der Oberseite (101a) eine flächige Unterseite (101b) aufweist,
wobei die Oberseite (101a) und die Unterseite (101b) durch mehrere Stirnseiten verbunden sind,
wobei die Öffnung (109) an einer der mehreren Stirnseiten angebracht ist.

7. MR-Lokalspule (100) nach einem der Ansprüche 4 bis 6, wobei die mehreren Stirnseiten eine umlaufend einheitliche Außennaht aufweisen.

8. MR-Lokalspule (100) nach Anspruch 7,
wobei die Außennaht eine Ultraschallschweißnaht ist.

9. MR-Lokalspule (100) nach einem der vorangehenden Ansprüche,
wobei der Rahmen (103) eine Führungsstruktur (107) aufweist, welche mit einer Gegenstruktur (106) der Antennenstruktur (104) so zusammenwirkt, dass die Antennenstruktur (104) im Innenraum der Außenhülle (101) auf Position gehalten wird.

10. MR-Lokalspule (100) nach einem der vorangehenden Ansprüche,
wobei die Antennenstruktur (104) eine Nut und/oder eine Zunge aufweist, durch welche die Antennenstruktur (104) innerhalb der Außenhülle (101) auf Position gehalten wird.

11. MR-Lokalspule (100) nach einem der vorangehenden Ansprüche,
wobei die MR-Lokalspule (100) einen Deckel (102) umfasst, der im Bereich des Rahmens (103) lösbar an der Außenhülle (101) befestigt ist.

12. MR-Lokalspule (100) nach Anspruch 11,
wobei der Rahmen (103) durch den Deckel (102) geklemmt wird.

13. Magnetresonanzvorrichtung (10) mit zumindest einer MR-Lokalspule nach einem der Ansprüche 1 bis 12.

14. Verfahren zur Herstellung einer MR-Lokalspule (100) umfassend
- eine Außenhülle (101), die flexibel ausgebildet ist und einen Innenraum umgibt,
- einen Rahmen (103) zum Aufnehmen einer Antennenstruktur (104), der zumindest bereichsweise starr ausgebildet und fest mit der Außenhülle (101) verbunden ist,
- eine Antennenstruktur (104) mit zumindest einer MR-Antenne, die in dem Innenraum der Außenhülle (101) angeordnet ist und von dem Rahmen (103) auf Position gehalten wird,
**dadurch gekennzeichnet, dass** der Rahmen an mehreren Sollbruchstellen gebrochen wird sodass der Rahmen mehrere resultierende Bruchstellen aufweist,
wobei die Antennenstruktur (104) entlang der Bruchstellen in Innenraum der Außenhülle (101) eingeführt wird.

15. Verfahren nach Anspruch 14 zur Herstellung einer MR-Lokalspule (100) nach einem der Ansprüche 2 bis 12.

## Claims

1. MR local coil (100), comprising
- an outer casing (101), which is embodied in a flexible manner and surrounds an inner area,
- a frame (103) for accommodating an antenna structure (104), which is embodied in a rigid manner, at least in regions, and is connected to the outer casing (101) in a fixed manner,
- an antenna structure (104) with at least one MR antenna, which is arranged in the inner area of the outer casing (101) and is held in position by the frame (103),
**characterised in that** the frame (103) has multiple break points resulting from a breaking of intended break points (108).

2. MR local coil (100) according to claim 1,
wherein the frame (103) is connected to the outer casing (101) by means of an ultrasonic welding seam.

3. MR local coil (100) according to one of the preceding claims,
wherein the outer casing (101) comprises an artificial leather and/or a PU-coated fabric.

4. MR local coil (100) according to one of the preceding claims,
wherein the outer casing (101) has an opening for introducing the MR antenna into the inner area of the outer casing.

5. MR local coil (100) according to claim 4,
wherein the frame (103) borders the opening (109).

6. MR local coil (100) according to claim 4 or 5,
wherein the outer casing (101) has a flat top side (101a), wherein the outer casing (101) has a flat bottom side (101b) opposite the top side (101a),
wherein the top side (101a) and the bottom side (101b) are connected by multiple end faces,
wherein the opening (109) is attached to one of the multiple end faces.

7. MR local coil (100) according to one of claims 4 to 6, wherein the multiple end faces have a circumferentially uniform outer seam.

8. MR local coil (100) according to claim 7,
wherein the outer seam is an ultrasonic welding seam.

9. MR local coil (100) according to one of the preceding claims,
wherein the frame (103) has a guide structure (107), which interacts with a matching structure (106) of the antenna structure (104) such that the antenna structure (104) is held in position in the inner area of the outer casing (101).

10. MR local coil (100) according to one of the preceding claims,
wherein the antenna structure (104) has a groove and/or a tongue, by way of which the antenna structure (104) is held in position within the outer casing (101).

11. MR local coil (100) according to one of the preceding claims,
wherein the MR local coil (100) comprises a cover (102), which is detachably fastened to the outer casing (101) in the region of the frame (103).

12. MR local coil (100) according to claim 11,
wherein the frame (103) is clamped by the cover (102).

13. Magnetic resonance apparatus (10) with at least one MR local coil according to one of claims 1 to 12.

14. Method for producing an MR local coil (100) comprising
- an outer casing (101), which is embodied in a flexible manner and surrounds an inner area,
- a frame (103) for accommodating an antenna structure (104), which is embodied in a rigid manner, at least in regions, and is connected to the outer casing (101) in a fixed manner,
- an antenna structure (104) with at least one MR antenna, which is arranged in the inner area of the outer casing (101) and is held in position by the frame (103),
**characterised in that**
the frame is broken at multiple intended break points, so that the frame has multiple resulting break points,
wherein the antenna structure (104) is introduced into the interior of the outer casing (101) along the break points.

15. Method according to claim 14 for producing an MR local coil (100) according to one of claims 2 to 12.

## Revendications

1. Bobine (100) locale RM comprenant,
- une enveloppe (101) extérieure, de constitution souple et entourant un espace intérieur,
- un cadre (103) de réception d'une structure (104) d'antenne, de constitution rigide au moins par endroits et relié fixement à l'enveloppe (101) extérieure,
- une structure (104) d'antenne ayant au moins une antenne RM, qui est disposée dans l'espace intérieur de l'enveloppe (101) extérieure et qui est maintenue en position par le cadre (103), **caractérisée en ce que** le cadre (103) a plusieurs points de rupture provenant d'une rupture de points (108) destinés à se rompre.

2. Bobine (100) locale RM suivant la revendication 1,
dans laquelle le cadre (103) est relié à l'enveloppe (101) extérieure au moyen d'un cordon de soudure par ultrasons.

3. Bobine (100) locale RM suivant l'une des revendications précédentes,
dans laquelle l'enveloppe (101) extérieure comprend un cuir artificiel et/ou un tissu revêtu de PU.

4. Bobine (100) locale RM suivant l'une des revendications précédentes,
dans laquelle l'enveloppe (101) extérieure a une ouverture d'introduction de l'antenne RM dans l'espace intérieur de l'enveloppe extérieure.

5. Bobine (100) locale RM suivant la revendication 4,
dans laquelle le cadre (103) est contigu à l'ouverture (109).

6. Bobine (100) locale RM suivant la revendication 4 ou 5, dans laquelle l'enveloppe (101) extérieure a un côté (101a) supérieur plat,
dans laquelle l'enveloppe (101) extérieure a un côté (101b) inférieur plat opposé au côté (101a) supérieur,
dans laquelle le côté (101a) supérieur et le côté (101b) inférieur sont reliés par plusieurs côtés frontaux,
dans laquelle l'ouverture (109) est ménagée sur l'un des plusieurs côtés frontaux.

7. Bobine (100) locale RM suivant l'une des revendications 4 à 6,
dans laquelle les plusieurs côtés frontaux ont un cordon extérieur unitaire faisant le tour.

8. Bobine (100) locale RM suivant la revendication 7,
dans laquelle le cordon extérieur est un cordon de soudure par ultrasons.

9. Bobine (100) locale RM suivant l'une des revendications précédentes,
dans laquelle le cadre (103) a une structure (107) de guidage, qui coopère avec une structure (106) antagoniste de la structure (104) d'antenne, de manière à maintenir la structure (104) d'antenne en position dans l'espace intérieur de l'enveloppe (101) extérieure.

10. Bobine (100) locale RM suivant l'une des revendications précédentes,
dans laquelle la structure (104) d'antenne a une rainure et/ou une languette, par laquelle la structure (104) d'antenne est maintenue en position à l'intérieur de l'enveloppe (101) extérieure.

11. Bobine (100) locale RM suivant l'une des revendications précédentes,
dans laquelle la bobine (100) locale RM comprend un couvercle (102) qui, dans la partie du cadre (103), est fixé de manière amovible à l'enveloppe (101) extérieure.

12. Bobine (100) locale RM suivant la revendication 11,
dans laquelle le cadre (103) est serré par le couvercle (102).

13. Dispositif (10) de résonnance magnétique comprenant au moins une bobine locale RM suivant l'une des revendications 1 à 12.

14. Procédé de fabrication d'une bobine (100) locale RM comprenant
- une enveloppe (101) extérieure, de constitution souple et entourant un espace intérieur,
- un cadre (103) de réception d'une structure (104) d'antenne, de constitution rigide au moins par endroits et relié fixement à l'enveloppe (101) extérieure,
- une structure (104) d'antenne ayant au moins une antenne RM, qui est disposée dans l'espace intérieur de l'enveloppe (101) extérieure et qui est maintenue en position par le cadre (103), **caractérisé**
**en ce que** l'on rompt le cadre en plusieurs points destinés à se rompre, de manière à ce que le cadre ait plusieurs points de rupture, qui s'ensuivent,
dans lequel on introduit la structure (104) d'antenne dans l'espace intérieur de l'enveloppe (101) extérieure le long des points de rupture.

15. Procédé suivant la revendication 14 de fabrication d'une bobine (100) locale RM suivant l'une des revendications 2 à 12.
